(19) Europäisches Patentamt
European Patent Office
Office européen des brevets

(11) EP 0 557 814 B1

(12) **EUROPÄISCHE PATENTSCHRIFT**

(45) Veröffentlichungstag und Bekanntmachung des Hinweises auf die Patenterteilung:
**01.05.1996 Patentblatt 1996/18**

(51) Int. Cl.$^6$: **B07C 5/342**, G01N 21/90

(21) Anmeldenummer: 93102208.1

(22) Anmeldetag: **12.02.1993**

(54) **Verfahren und Vorrichtung zur selektiven Identifikation und Unterscheidung von Schadstoffen in Abfüllanlagen der Getränkeindustrie**

Method and apparatus for selective identification and separation of contaminants in filling plants for the beverage industry

Procédé et dispositif pour l'identification et la distinction sélectives de substances nocives dans des installations de remplissage pour l'industrie des boissons

(84) Benannte Vertragsstaaten:
**AT BE CH DE DK ES FR GB GR IE IT LI NL PT SE**

(30) Priorität: **25.02.1992 DE 4205722**

(43) Veröffentlichungstag der Anmeldung:
**01.09.1993 Patentblatt 1993/35**

(73) Patentinhaber: **Krieg, Gunther, Prof.Dr.Ing.**
**D-76227 Karlsruhe (DE)**

(72) Erfinder:
• **Koukolitschek, Karl, Dipl.-Ing.**
**W-7500 Karlsruhe 21 (DE)**

• **Maier, Wilfried**
**W-7519 Sulzfeld (DE)**
• **Krieg, Gunther, Prof.Dr.Ing.**
**W-7500 Karlsruhe 41 (DE)**

(74) Vertreter: **Dipl.-Ing. Heiner Lichti**
**Dipl.-Phys. Dr.rer.nat. Jost Lempert**
**Dipl.-Ing. Hartmut Lasch**
**Postfach 41 07 60**
**D-76207 Karlsruhe (DE)**

(56) Entgegenhaltungen:
**WO-A-88/00862        WO-A-89/09931**

**Beschreibung**

Die Erfindung betrifft ein Verfahren nach dem Oberbegriff des Anspruchs 1 und eine Vorrichtung nach dem Oberbegriff des Anspruchs 11.

Neben der in der nicht vorveröffentlichten US 5,305,887 und der deutschen Patentanmeldung P 4203274.1 gelösten Problemstellung der Schadstoffidentifikation und Schadstoffklassifizierung, verbunden mit der Aussortierung von mit Schadstoffen kontaminierten Flaschen/Behältern, z.B. in Abfüllinien, spielt insbesondere der Wirtschaftlichkeitsaspekt eine große Rolle. Dieser Aspekt verlangt, daß die eigentlichen, zur jeweiligen Mehrwegflasche/zum jeweiligen Mehrwegbehälter gehörigen Produkte nicht zu einer Ausschleusung führen. In der Getränkeindustrie bedeutet dies, daß z.B. mit Benzin oder Diesel etc. kontaminierte Flaschen/Behälter aussortiert werden, daß jedoch Getränkeinhaltsstoffe, wie z.B. Zitrusaromen oder unschädliche Gärprodukte, wie z.B. Ethanol, nicht zu einer Ausschleusung führen. In der Praxis folgt daraus, daß beispielsweise der Inhaltsstoff Lemonen, der teilweise in höheren Konzentrationen in Süßgetränken enthalten ist, als "gut"-Stoff erkannt wird. Entsprechendes gilt für Ethanol, solange die Konzentrationen entsprechend den ablaufenden Gärprozessen relativ niedrig sind.

Die Anwesenheit von Ethanol muß aber dann zur Ausschleusung führen, wenn die Konzentrationen so hoch sind, daß nicht auszuschließen ist, daß es sich nicht um reine Gärprodukte, sondern um Waschmittel oder Spiritus handelt, die gleichermaßen zu Geschmacksverfälschungen bei Wiederbefüllung der Flasche/des Behälters mit neuem Produkt führen können. Entsprechendes gilt für eine Kontamination mit Reinigungsmitteln, die oft aus Gründen der Geruchskaschierung mit hohen Prozentsätzen an Lemonen versetzt werden und aufgrund der Bestandteile an Reinigungschemikalien auszusortieren sind.

Die WO 89/09931 zeigt ein Verfahren zur Identifikation von Flüssigkeiten in Flaschen oder dergleichen, nach dem die Summe der Intensitäten für eine Kontrollflüssigkeit und eine Testflüssigkeit ermittelt und die Intensitäten anschließend verglichen werden oder aber die Abweichung zwischen entsprechenden Test- und Kontrolldaten ermittelt und dann festgestellt wird, ob die Abweichung zu groß ist.

Die WO-A-88/00862 zeigt Verfahren zur Feststellung von kontaminierten und unkontaminierten Behältnissen, die keine zufriedenstellenden Resultate im Hinblick auf die vorgenannte Problematik ergeben.

Der Erfindung liegt die Aufgabe zugrunde, ein Verfahren und eine Vorrichtung anzugeben, bei denen ein Stoff, der unterhalb einer vorgegebenen Konzentration nicht zu beanstanden ist, dann zu einer Ausschleusung eines ihn enthaltenden Behälters führt, wenn seine Konzentration oberhalb der vorgebenen Konzentration liegt.

Zur Lösung der Aufgabe sieht die Erfindung ein Verfahren und eine Vorrichtung der eingangs genannten Art vor, die weiterhin die kennzeichnenden Merkmale der Ansprüche 1 bzw. 11 aufweisen.

In bevorzugter Ausgestaltung ist vorgesehen, daß die durch spezielle "Fingerprints" gekennzeichneten "gut"- und "schlecht"-Stoffe gemäß der US 5,305,887 jeweils als zu der einen oder anderen Kategorie zugehörig erkannt werden und daß als Kriterium für die Nicht-Ausschleusung bzw. Ausschleusung Schwellen eingeführt werden. Diese Schwellen werden so gewählt daß bei Konzentrationen ober- oder unterhalb vorzuwählender Konzentrationen eine Ausschleusung bzw. keine Ausschleusung erfolgt.

Im speziellen Fall von Zitrusaromen, z.B. von Lemonen, bedeutet dies, daß erfindungsgemäß zunächst die Zitruskomponente als "gut"-Stoff erkannt wird und daß in einem nächsten Verfahrensschritt geprüft wird, ob die Konzentration unterhalb einer Schwelle $S_1$ liegt, die z.B. so gewählt wird, daß sie für Süßgetränke aller Art nicht überschritten wird und nicht zu einer Ausschleusung führt.

Sollte die Lemonen-Konzentration jedoch oberhalb von $S_2$ liegen, so wird erfindungsgemäß zusätzlich geprüft, ob z.B. mit Lemonen versetzte Reinigungsmittel vorliegen. Sobald eine Schwelle $S_2$ überschritten wird, die unterhalb der Lemonen-Konzentration gängiger Reinigungsmittel liegt, wird die betreffende Flasche/Behälter entsprechend der vorliegenden Erfindung ausgeschieden. Entsprechendes gilt z.B. für Ethanol, der beispielsweise bei der Gärung von Süßgetränken in kleineren Konzentrationen entsteht und wegen der Unterschreitung der Schwelle $S_3$ in der Getränke-Abfüllinie verbleibt, wohingegen bei Überschreiten der Schwelle $S_4$ eine Ausschleusung erfolgt, da z.B. Spiritus, hochkonzentrierte Spirituosen, Wein usw. als ethanolhaltige, geschmacksverfälschende Stoffe vorliegen können.

Um die erfindungsgemäß vorgesehene Klassifizierung in die Bestandteilgruppen:

- "echte Schadstoffe", wie z.B. Aceton, Benzin, Diesel, Methanol, Xylol, Benzol, Toluol etc. und Gemische derselben

- "getarnte Schadstoffe", wie z.B. mit Lemonen versetzte Reiniger, mit Ethanol versetzte Waschmittel etc.

- "Flaschen-/ Behälter-spezifische Produkte" wie z.B. Süßgetränke, Mineralwässer, Fruchtsäfte etc.

mit an Sicherheit grenzender Wahrscheinlichkeit durchführen zu können, sind erfindungsgemäß wirksame Maßnahmen zur selektiven Stofferkennung vorgesehen. Darüber hinaus werden Verfahren und Vorrichtungen für die Konzentrationsbestimmung der oben genannten Stoffkomponenten, d.h. für die präzise Einordnung in unterhalb der Schwelle $S_1$, $S_3$

2

EP 0 557 814 B1

liegende "Flaschen- Behälter- spezifische Produkte" einschließlich deren Gärprodukte und oberhalb der Schwelle $S_2$, $S_4$ liegende "echte"- bzw. "getarnte"- Schadstoffe, erfindungsgemäß vorgeschlagen.

Aufbauend auf der technischen Lehre von US 905,470, wird die Schadstofferkennung erfindungsgemäß entscheidend verbessert, indem die durch Spektralverfahren im ultravioletten-, sichtbaren-, infraroten- und Mikrowellen- Spektralbereich vorliegenden stoffspezifischen Spektren, einem neuartigen Auswerteverfahren unterzogen werden. Das neue Verfahren beruht darauf, daß der Vergleich der aktuell an einer Flaschen-/Behälterprobe gemessenen Spektren mit in einem Speicher abgelegten Spektren derart erfolgt, daß gemäß der Erfindung verglichen wird, ob aktuell gemessene und gespeicherte Spektren übereinstimmen in:

- den <u>Positionen der Maxima und Minima</u> auf der Wellenlängenskala

- den <u>Halbwertsbreiten</u>, d.h. den Wellenlängenintervallen auf halber Höhe

- den <u>Fußbreiten</u>, d.h. den Wellenlängenintervallen, innerhalb derer ein Spektrum vorliegt

- den <u>Amplitudenverhältnissen</u> der vorliegenden <u>Maxima</u>

- den <u>Amplitudenverhältnissen</u> der vorliegenden <u>Minima</u>

- den <u>Amplitudenverhältnissen</u> von <u>Maxima</u> <u>und</u> <u>Minima</u>

Durch diese wirksamen Maßnahmen wird insbesondere sichergestellt, daß die in der Getränkeindustrie vorliegenden hohen Flaschenleistungen von bis zu 50.000 Flaschen pro Stunde mit einem einzigen Sensorsystem bei gleichzeitig wirtschaftlich vertretbarem Rechenaufwand, d.h. stark eingeschränkter Rechenleistung, die geforderte on-line- Auswertung erstmals realisiert werden kann.

Da die Fehlausleitung von Flaschen, d.h. die Ausleitung ohne Notwendigkeit, in der Getränkeindustrie manuelle Zusatzarbeiten erforderlich macht, die zu unzulässigen Zusatzkosten führen, sind zwecks Vermeidung dieser Kosten erfindungsgemäß weitere Maßnahmen vorgesehen, die im Zusammenwirken mit den oben genannten Maßnahmen die Sicherheit der selektiven Stofferkennung auf nahezu 100,0 % steigern. Dazu werden im Rahmen der Erfindung die Spektren punktpaarweise abgetastet und folgende Verfahren im Rahmen des Vergleichs zwischen aktuell gemessenen und abgespeicherten Spektren angewandt;

- Ermittlung der Summe der quadratischen Abweichungen aller Einzelpunkte.

- Ermittlung der Summe der Differenzen aller Einzelpunkte.

Dabei gilt derjenige Stoff innerhalb der Vielzahl gespeicherter Stoffe als erkannt, bei dem die obigen Größen ein Minimum aufweisen und bei dem das jeweilige Maximum unterhalb einer vom Betreiber der Anlage einzustellenden Zahl liegt.

In einer weiteren Ausbildung der Erfindung werden die Quotienten zwischen dem aktuellen und den gespeicherten Spektren gebildet und die jeweils resultierende Quotientenfunktion ausgewertet.

Die Erfindung wird im folgenden durch die Figuren 1 bis 6 näher beschrieben. Dabei zeigen:

Fig. 1: Die unterschiedlichen Konzentrationen von LEMONEN in einem Süßgetränk bzw. einem Haushaltsreiniger mit den Schwellen $S_3$ und $S_4$.

Fig. 2: Die unterschiedlichen Konzentrationen von ETHAHOL in einem gegorenen Süßgetränk bzw. einer hochprozentigen Spirituose mit den Schwellen $S_3$ und $S_4$.

Fig. 3: Die selektive Stoffidentifikation über ausgewählte Spektrenparameter

Fig. 4: Die selektive Stoffidentifikation über die Summe der quadratischen Abweichungen und die selektive Stoffidentifikation über die Summe der Differenzen.

Fig.5a: Die Quotientenbildung zwischen Spektren verschiedener Stoffe.

Fig.5b: Die Quotientenbildung zwischen Spektren identischer Stoffe.

Fig. 6: Ein Schema der Gesamtanordnung des erfindungsgemäßen Systems

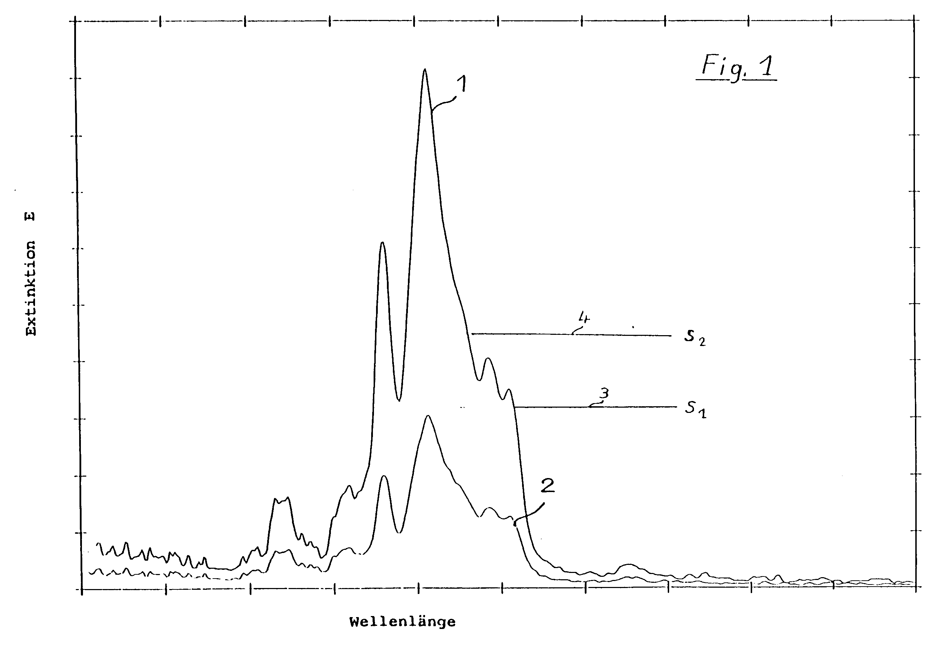

Fig. 1 zeigt eine mit einer Anordnung gemäß US 905,470 erzielte spektrale Verteilung der gasförmigen Phase des Stoffes Lemonen in einem Allzweckreiniger (1) und einem Süßgetränk (2). Man sieht, daß die Konzentration von Lemonen in dem Allzweckreiniger (1) wesentlich höher ist, als die im Süßgetränk (2). Durch die erfindungsgemäße Einführung der Schwellen $S_1$ (3) und $S_2$ (4) können mit Lemonen getarnte Schadstoffe (1) ausgeschleust werden, wohingegen mit Lemonen versetzte Süßgetränke (2) in der Flasche/ Behälter verbleiben. Die Schwelle $S_1$ (3) wird dabei so gelegt, daß sie über den Lemonen-Konzentrationen aller Süßgetränke liegt.

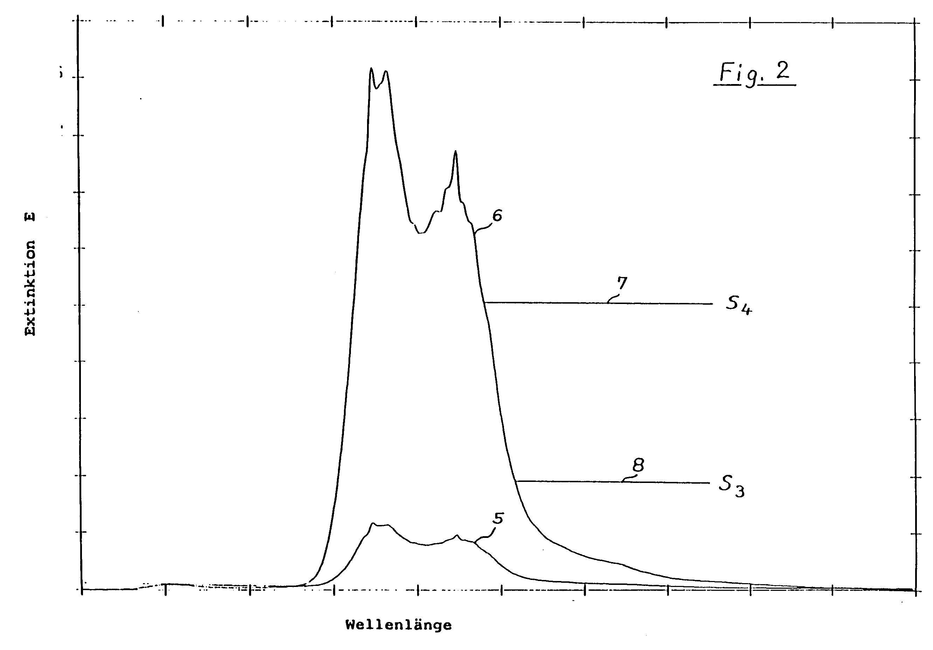

Die Schwelle $S_2$ (4) wird so festgesetzt, daß die Lemonen-Konzentration der betreffenden Reinigungsmittel (1) etc., oberhalb der Schwelle $S_2$ (4) liegen. Entsprechendes gilt für die in Fig. 2: gezeigten Ethanolkonzentrationen eines vergorenen Süßgetränkes (5) und einer hochprozentigen Spirituose (6). Da die Spirituose über der Schwelle $S_4$ (7) liegt, wird die zugeordnete Flasche ausgeschleust. Demgegenüber verbleibt die Flasche mit gegorenem Süßgetränk in der Abfüllinie, da die maximale Ethanolkonzentration (5) unterhalb der Schwelle $S_3$ (8) liegt.

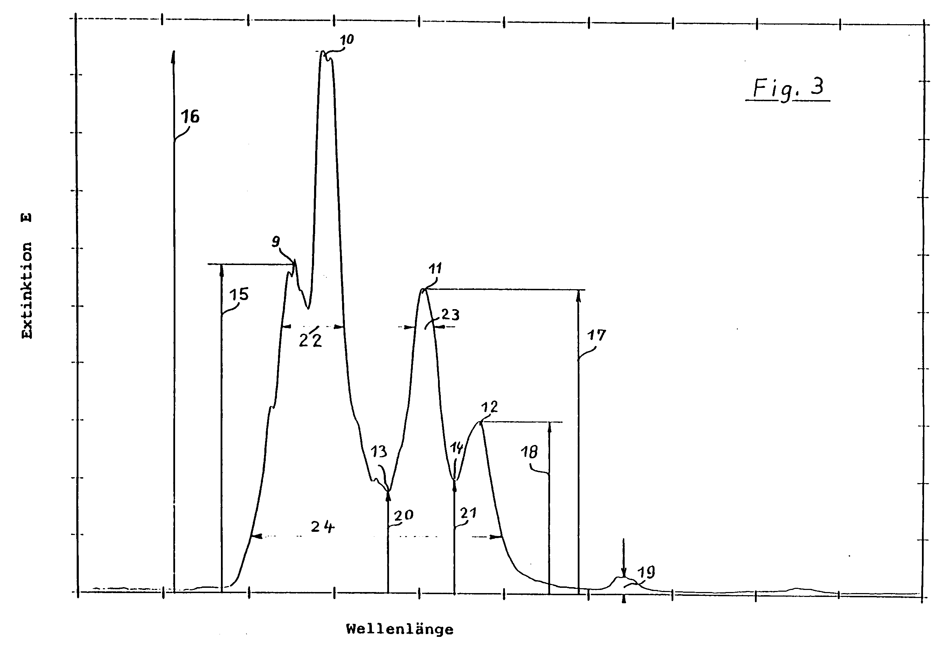

Fig. 3 zeigt die erfindungsgemäße Stoffidentifikation über ausgewählte Spektrenparameter am Beispiel des Schadstoffs Pentachlorphenol. Zu diesem Zweck wird zunächst überprüft, welcher Stoff, der in einer Bibliothek enthalten ist, Maximas (9,10,11,12) bzw. Minimas (13,14) bei denselben Wellenlängen wie der aktuell erfaßte Stoff aufweist. Danach wird überprüft, ob die Verhältnisse der relativen Maximas (15,16,17, 18,19), d.h. (15):(16), (15):(17), (15):(18), (15):(19), (16):(17), (16):(18), (16):(19), (17):(18), (17):(19), (18):(19) in gewissen vorgebbaren Grenzen mit den im Speicher abgelegten Quotienten übereinstimmen. Schließlich wird geprüft, ob die Verhältnisse der Amplituden (20,21) der Minimas, d.h. (20):(21) in den vorgegebenen Grenzen mit den gespeicherten Quotienten übereinstimmen. Zur weiteren Erhöhung der Sicherheit der Identifikation können die entsprechenden Verhältnisse aus den minimalen- und den maximalen Amplitudenwerten gebildet werden. Weitere charakteristische Merkmale sind den Halbwertsbreiten (22,23) sowie der Fußbreite (24), z.B. bei 10% der Maximalamplitude, zu entnehmen.

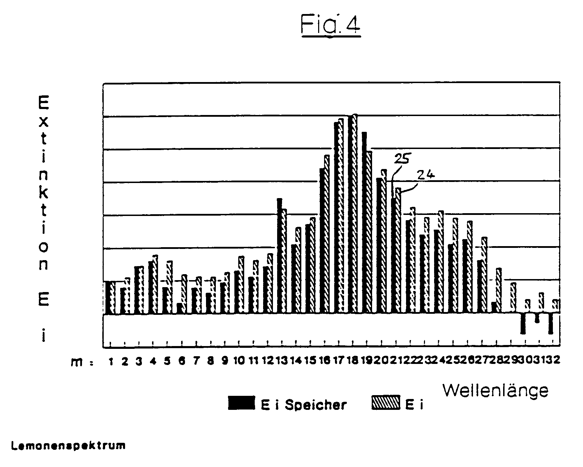

Nach der Erfindung wird gemäß Figur 4 die Summe der quadratischen Abweichungen bzw. die Summe der Absolutwerte der Differenzen zwischen dem aktuellen Spektrum (24) und den im Speicher abgelegten Spektren (25) gebildet. Fig. 4 zeigt dazu als Beispiel ein Spektrum (25) von Lemonen aus dem Speicher im Vergleich zu einem aktuell gemessenen Lemonenspektrum (24). Die Bildung der

Summe der quadratischen Abweichungen

$$\sum_{i=1}^{m} (E_{i\,Speicher} - E_i)^2 \text{ bzw. von der}$$

Summe der Differenzen

$$\sum_{i=1}^{m} |(E_{i\,Speicher} - E_i)|$$

beider Spektren führt in beiden Fällen zu Minimalwerten der Summen im Vergleich zu den Fällen, bei denen Spektren unterschiedlicher Stoffe verglichen werden.
Dabei ist:

$E_i =$ $\alpha_i \cdot l \cdot n_i =$ Extinktion
$\alpha_i =$ Absorptionkoeffizient
$l =$ Optische Weglänge
$n_i =$ Anzahl der Schadstoffmoleküle/cm$^3$

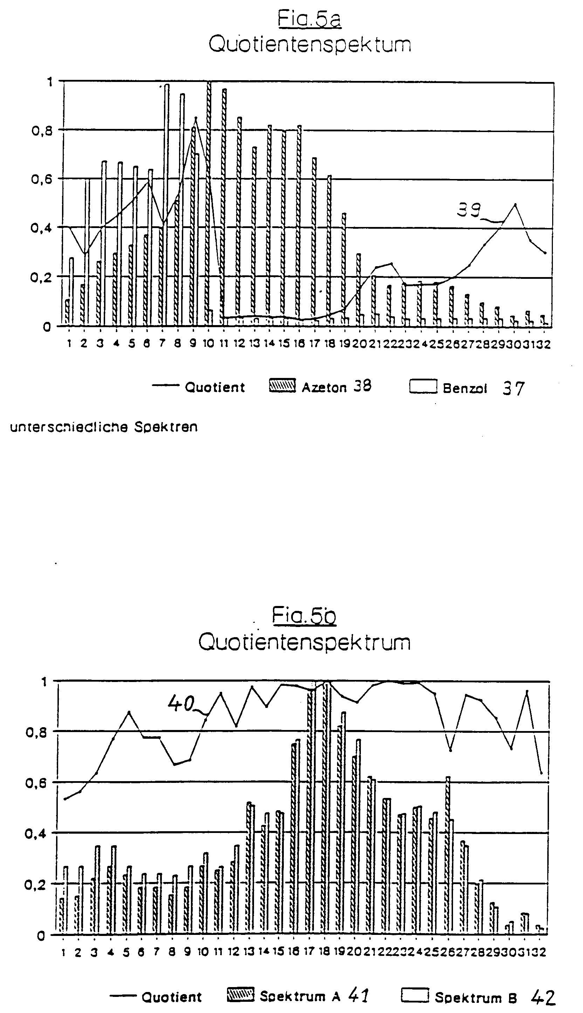

Fig. 5a zeigt das Ergebnis der Quotientenbildung (39) zwischen Spektren verschiedener Stoffe, in diesem Fall von Benzol (37) und Aceton (38), Fig, 5b gibt das entsprechende Ergebnis der Quotientenbildung von einem Spektrum aus dem Speicher (41) und einem aktuellen Spektrum (42) für jeweils gleiche Stoffe, wie z.B. Lemonen, wieder. Zur einfachen Auswertung wird erfindungsgemäß jeweils die Gesamtsumme der Einzelwerte des Quotientenspektrums gebildet, wobei die maximale Summe den identischen Stoff charakterisiert.

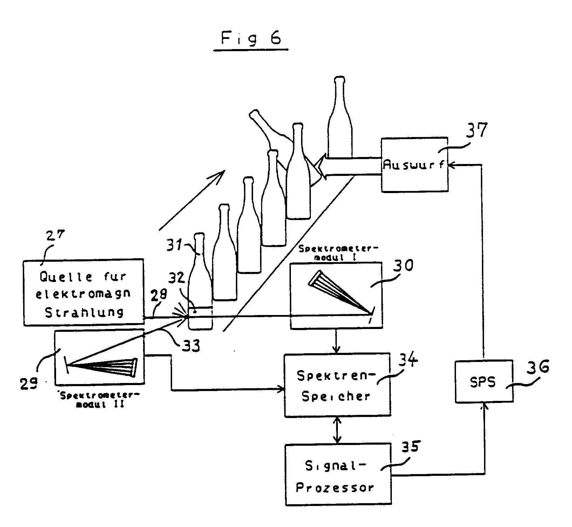

Das erfindungsgemäße Gesamtsystem ist in Fig. 6 dargestellt. Eine Quelle (27) zur Erzeugung elektromagnetischer Strahlung im ultravioletten-, infraroten-, Mikrowellen-Bereich durchstrahlt die zu untersuchende Flasche (31)/bzw. den Behälter gemäß dem Strahlverlauf (28) bzw. gemäß dem Strahlverlauf des gestreuten bzw. reflektierten Strahls (33). Die von den Inhaltsstoffen (32) bzw. Schadstoffen (32) bewirkten Veränderungen in der spektralen Zusammensetzung der Strahlung werden von den Spektrometermodulen I, II (30,29) analysiert. Die resultierenden Spektren werden von einem Signalprozessor (35) mit den in einem Spektrenspeicher (34) gespeicherten, bekannten Spektren on-line verglichen. Falls die Randbedingungen für eine Flaschen-/Behälter-Ausschleusung erfüllt sind, wird über einen Befehl an die Maschinensteuerung (36), die Ausschleusungseinrichtung (37) aktiviert und die Flasche ausgeschleust.

**Patentansprüche**

1. Verfahren zur Identifikation und Unterscheidung zwischen Schadstoffen und Inhaltsstoffen in Flaschen und Behältern unter Einsatz der Absorption, Reflexion und Streuung elektromagnetischer Wellen von UV- bis in den Mikrowellenbereich durch Auswertung stoffspezifischer Spektren, dadurch gekennzeichnet, daß die Summen positiver Funktionen der Differenzen zwischen zugeordneten Ordinatenwerten der aktuell on-line gemessenen und im Speicher befindlichen bekannten Spektren gebildet wird und der Stoff als erkannt gilt, der den kleinsten Summenwert aufweist.

2. Verfahren nach Anspruch 1, dadurch gekennzeichnet, daß die Summe der quadratischen Abweichungen zwischen zugeordneten Ordinatenwerten der aktuell on-line gemessenen und der im Speicher befindlichen Spektren gebildet wird und der Stoff als erkannt gilt, der den kleinsten Summenwert aufweist.

3. Verfahren nach Anspruch 1, dadurch gekennzeichnet, daß die Summe der Differenz zwischen zugeordneten Ordinatenwerten der aktuell on-line gemessenen und der im Speicher befindlichen Spektren gebildet wird und der Stoff als erkannt gilt, der den kleinsten Summenwert aufweist.

4. Verfahren nach einem der Ansprüche 1 bis 3, dadurch gekennzeichnet, daß die Positionen der relativen Amplitudenmaxima auf der Wellenlängenskala zur on-line-Identifikation herangezogen werden.

5. Verfahren nach einem der Ansprüche 1 bis 4, dadurch gekennzeichnet, daß die Positionen der relativen Amplitudenminima auf der Wellenlängenskala zur on-line-Identifikation herangezogen werden.

6. Verfahren nach einem der vorangehenden Ansprüche, dadurch gekennzeichnet, daß die Positionen der relativen Amplitudenmaxima und -minima auf der Wellenlängenskala zur on-line-Identifikation der Inhaltsstoffe herangezogen werden.

7. Verfahren nach einem der Ansprüche 4 bis 6, dadurch gekennzeichnet, daß die Verhältnisse der Amplituden der Maxima, der Minima und zwischen Maxima und Minima on-line gebildet werden.

8. Verfahren nach einem der Ansprüche 1 bis 7, dadurch gekennzeichnet, daß die Halbwertsbreiten bzw. Fußbreiten der Spektren zur Identifikation und Unterscheidung herangezogen werden.

9. Verfahren nach einem der Ansprüche 1 bis 8, dadurch gekennzeichnet, daß die Summe der Quotienten zwischen zugeordneten Ordinatenwerten der aktuell on-line gemessenen und der im Speicher befindlichen Spektren gebildet wird und der Stoff als erkannt gilt, der den größten Summenwert aufweist.

10. Verfahren nach einem der Ansprüche 1 bis 9, dadurch gekennzeichnet, daß eine erste Schwelle für die Maximalamplitude gewählt wird, unterhalb der Behälter-/Flaschen-Inhaltsstoffe in der Abfüllinie verbleiben, wohingegen inhaltsidentische Stoffe zu einer Ausschleusung führen, sobald eine zweite Schwelle, die oberhalb der ersten liegt, überschritten wird.

11. Vorrichtung zur Identifikation und Unterscheidung zwischen Schadstoffen und Inhaltsstoffen in Flaschen und Behältern unter Einsatz der Absorption, Reflexion und Streuung elektromagnetischer Wellen von UV- bis in den Mikrowellenbereich durch Auswertung stoffspezifischer Spektren, dadurch gekennzeichnet, daß die Summe positiver Funktionen der Differenzen zwischen zugeordneten Ordinatenwerten (24,25) der aktuell on-line gemessenen und im Speicher (34) befindlichen bekannten Spektren (1,2,5,6) gebildet wird und der Stoff (32) als erkannt gilt, der den kleinsten Summenwert aufweist.

12. Vorrichtung nach Anspruch 11, dadurch gekennzeichnet, daß die Summe der quadratischen Abweichungen zwischen zugeordneten Ordinatenwerten (24,25) der aktuell on-line gemessenen und der im Speicher (34) befindlichen Spektren (1,2,5,6) gebildet wird und der Stoff (32) als erkannt gilt, der den kleinsten Summenwert aufweist.

13. Vorrichtung nach Anspruch 11, dadurch gekennzeichnet, daß die Summe der Differenz zwischen zugeordneten Ordinatenwerten (24,25) der aktuell on-line gemessenen und der im Speicher befindlichen Spektren (1,2, 5,6) gebildet wird und der Stoff als erkannt gilt, der den kleinsten Summenwert aufweist.

14. Vorrichtung nach einem der Ansprüche 11 bis 13, dadurch gekennzeichnet, daß die Positionen der relativen Amplitudenmaxima auf der Wellenlängenskala zur on-line-Identifikation herangezogen werden.

**15.** Vorrichtung nach einem der Ansprüche 11 bis 13, dadurch gekennzeichnet, daß die Positionen der relativen Amplitudenminima auf der Wellenlängenskala zur on-line-Identifikation herangezogen werden.

**16.** Vorrichtung nach einem der Ansprüche 11 bis 15, dadurch gekennzeichnet, daß die Positionen der relativen Amplitudenmaxima und -minima auf der Wellenlängenskala zur on-line-Identifikation der Inhaltsstoffe herangezogen werden.

**17.** Vorrichtung nach einem der Ansprüche 11 bis 16, dadurch gekennzeichnet, daß die Verhältnisse der Amplituden der Maxima, der Minima und zwischen Maxima und Minima on-line gebildet werden.

**18.** Vorrichtung nach einem der Ansprüche 11 bis 17, dadurch gekennzeichnet, daß die Halbwertsbreiten (22,23) bzw. Fußbreiten (24) der Spektren (1,2,5,6) zur Identifikation und Unterscheidung herangezogen werden.

**19.** Vorrichtung nach einem der Ansprüche 11 bis 18, dadurch gekennzeichnet, daß die Summe der Quotienten (39,40) zwischen zugeordneten Ordinatenwerten (37,38, 41,42) der aktuell on-line gemessenen und der im Speicher befindlichen Spektren (1,2,5,6) gebildet wird und der Stoff als erkannt gilt, der den größten Summenwert aufweist.

**20.** Vorrichtung nach einem der Ansprüche 11 bis 19, dadurch gekennzeichnet, daß eine erste Schwelle (3,8) für die Maximalamplitude gewählt wird, unterhalb der Behälter-/Flaschen-Inhaltsstoffe in der Abfüllinie verbleiben, wohingegen inhaltsidentische Stoffe zu einer Ausschleusung führen, sobald eine zweite Schwelle (4,7), die oberhalb der ersten liegt, überschritten wird.

**Claims**

**1.** Process for identifying and distinguishing between contaminating substances and content substances in bottles and containers using the absorption, reflection and scattering of electromagnetic waves from the UV to the microwave range by evaluating substance-specific spectra, characterized in that the sums of positive functions of the differences between associated ordinate values of the actual, on-line measured spectra and the known spectra in the memory are formed and the substance having the lowest sum value is considered to be identified.

**2.** Process according to claim 1, characterized in that the sum is formed of the standard deviations between associated ordinate values of the actual, on-line measured spectra and the spectra in the memory and the substance having the lowest sum value is considered to be identified.

**3.** Process according to claim 1, characterized in that the sum of the difference between associated ordinate values of the actual, on-line measured spectra and the spectra in the memory is formed and the substance having the lowest sum value is considered to be identified.

**4.** Process according to one of the claims 1 to 3, characterized in that the positions of the relative amplitude maxima on the wavelength scale are used for on-line identification.

**5.** Process according to one of the claims 1 to 4, characterized in that the positions of the relative amplitude minima on the wavelength scale are used for on-line identification.

**6.** Process according to one of the preceding claims, characterized in that the positions of the relative amplitude maxima and minima on the wavelength scale are used for the on-line identification of the content substances.

**7.** Process according to one of the claims 4 to 6, characterized in that the ratios of the amplitudes of the maxima, minima and between the maxima and minima are formed on-line.

**8.** Process according to one of the claims 1 to 7, characterized in that the half-widths or base widths of the spectra are used for identification and distinguishing.

**9.** Process according to one of the claims 1 to 8, characterized in that the sum of the quotients between associated ordinate values of the actual, on-line measured spectra and those in the memory is formed and the substance having the highest sum value is considered to be identified.

10. Process according to one of the claims 1 to 9, characterized in that a first threshold is chosen for the maximum amplitude below which the container/bottle content substances remain in the filling line, whilst content-identical substances lead to a discharge on exceeding a second threshold, which is above the first.

11. Apparatus for identifying and distinguishing between contaminating substances and content substances in bottles and containers using the absorption, reflection and scattering of electromagnetic waves from the UV to the microwave range by evaluating substance-specific spectra, characterized in that the sum of positive functions of the differences between associated ordinate values (24, 25) of the actual, on-line measured spectra (1, 2, 5, 6) and those in the memory (34) is formed and the substance (32) having the smallest sum value is considered to be identified.

12. Apparatus according to claim 11, characterized in that the sum of the standard deviations between the associated ordinate values (24, 25) of the actual, on-line measured spectra (1, 2, 5, 6) and those in the memory (34) is formed and the substance (32) having the smallest sum value is considered to be identified.

13. Apparatus according to claim 11, characterized in that the sum of the difference between associated ordinate values (24, 25) of the actual, on-line measured spectra (1, 2, 5, 6) and those in the memory is formed and the substance having the smallest sum value is considered to be identified.

14. Apparatus according to one of the claims 11 to 13, characterized in that the positions of the relative amplitude maxima on the wavelength scale is used for on-line identification.

15. Apparatus according to one of the claims 11 to 13, characterized in that the positions of the relative amplitude minima on the wavelength scale are used for on-line identification.

16. Apparatus according to one of the claims 11 to 15, characterized in that the positions of the relative amplitude maxima and minima on the wavelength scale are used for on-line identification of the content substances.

17. Apparatus according to one of the claims 11 to 16, characterized in that the ratios of the amplitudes of the maxima, minima and between the maxima and minima are formed on-line.

18. Apparatus according to one of the claims 11 to 17, characterized in that the half-widths (22, 23) or base widths (24) of the spectra (1, 2, 5, 6) are used for identification and distinguishing.

19. Apparatus according to one of the claims 11 to 18, characterized in that the sum of the quotients (39, 40) between the associated ordinate values (37, 38, 41, 42) of the actual, on-line measured spectra (1, 2, 5, 6) and those in the memory is formed and the substance having the highest sum value is considered as identified.

20. Apparatus according to one of the claims 11 to 19, characterized in that a first threshold (3, 8) is chosen for the maximum amplitude below which container/bottle content substances remain in the filling line, whereas content-identical substances lead to a discharge as soon as a second threshold (4, 7) which is above the first, is exceeded.

**Revendications**

1. Procédé pour la distinction entre des substances nocives et des constituants dans des bouteilles et dans des récipients, ainsi que pour leur identification, par la mise en oeuvre de l'absorption, de la réflexion et de la dispersion d'ondes électromagnétiques du domaine des UV au domaine des micro-ondes, par évaluation de spectres spécifiques à des substances, caractérisé en ce qu'on fait la somme de fonctions positives des différences entre des valeurs de coordonnées correspondantes des spectres mesurés en ligne in situ et des spectres connus mis en mémoire, et la substance qui présente la plus petite valeur cumulée est considérée comme étant reconnue.

2. Procédé selon la revendication 1, caractérisé en ce qu'on fait la somme des déviations quadratiques entre des valeurs de coordonnées correspondantes des spectres mesurés en ligne in situ et les spectres se trouvant dans la mémoire, et la substance qui présente la plus petite valeur cumulée est considérée comme étant reconnue.

3. Procédé selon la revendication 1, caractérisé en ce qu'on fait la somme de la différence entre des valeurs de coordonnées correspondantes des spectres mesurés en ligne in situ et les spectres se trouvant dans la mémoire, et la substance qui présente la plus petite valeur cumulée est considérée comme étant reconnue.

**4.** Procédé selon l'une quelconque des revendications 1 à 3, caractérisé en ce que, pour l'identification en ligne, on part des positions des maxima d'amplitude relatifs sur l'échelle des longueurs d'ondes.

**5.** Procédé selon l'une quelconque des revendications 1 à 4, caractérisé en ce que, pour l'identification en ligne, on part des positions des minima d'amplitude relatifs sur l'échelle des longueurs d'ondes.

**6.** Procédé selon l'une quelconque des revendications précédentes, caractérisé en ce que, pour l'identification en ligne des constituants, on part des positions des maxima et des minima d'amplitude relatifs sur l'échelle des longueurs d'ondes.

**7.** Procédé selon l'une quelconque des revendications 4 à 6, caractérisé en ce qu'on calcule en ligne les rapports des amplitudes des maxima, des minima et entre les maxima et les minima.

**8.** Procédé selon l'une quelconque des revendications 1 à 7, caractérisé en ce que, pour l'identification et la distinction, on part des largeurs de demi-amplitudes, respectivement des largeurs de base des spectres.

**9.** Procédé selon l'une quelconque des revendications 1 à 8, caractérisé en ce qu'on fait la somme des quotients entre les valeurs de coordonnées correspondantes des spectres mesurés en ligne in situ et des spectres se trouvant dans la mémoire, et la substance qui présente la plus grande valeur cumulée est considérée comme étant reconnue.

**10.** Procédé selon l'une quelconque des revendications 1 à 9, caractérisé en ce qu'on sélectionne un premier seuil pour l'amplitude maximale en dessous duquel les constituants des récipients/bouteilles restent dans la ligne de transvasement, tandis que des substances identiques quant à leur contenu sont guidées vers un éclusage au-dehors dès qu'un second seuil qui se situe au-dessus du premier est dépassé.

**11.** Dispositif pour la distinction entre des substances nocives et des constituants dans des bouteilles et des récipients, ainsi que pour leur identification, par la mise en oeuvre de l'absorption, de la réflexion et de la dispersion d'ondes électromagnétiques du domaine des UV au domaine des micro-ondes, par évaluation de spectres spécifiques à des substances, caractérisé en ce qu'on fait la somme de fonctions positives des différences entre des valeurs de coordonnées correspondantes (24, 25) des spectres mesurés en ligne in situ et des spectres connus (1, 2, 5, 6) mis en mémoire (34), et la substance (32) qui présente la plus petite valeur cumulée est considérée comme étant reconnue.

**12.** Dispositif selon la revendication 11, caractérisé en ce qu'on fait la somme des déviations quadratiques entre des valeurs de coordonnées correspondantes (24, 25) des spectres mesurée en ligne in situ et les spectres (1, 2, 5, 6) mis en mémoire (34), et la substance (32) qui présente la plus petite valeur cumulée est considérée comme étant reconnue.

**13.** Dispositif selon la revendication 11, caractérisé en ce qu'on fait la somme de la différence entre des valeurs de coordonnées correspondantes (24, 25) des spectres mesurés en ligne in situ et les spectres (1, 2, 5, 6) mis en mémoire, et la substance qui présente la plus petite valeur cumulée est considérée comme étant reconnue.

**14.** Dispositif selon l'une quelconque des revendications 11 à 13, caractérisé en ce que, pour l'identification en ligne, on part des positions des maxima d'amplitude relatifs sur l'échelle des longueurs d'ondes.

**15.** Dispositif selon l'une quelconque des revendications 11 à 13, caractérisé en ce que, pour l'identification en ligne, on part des positions des minima d'amplitude relatifs sur l'échelle des longueurs d'ondes.

**16.** Dispositif selon l'une quelconque des revendications 11 à 15, caractérisé en ce que, pour l'identification en ligne des constituants, on part des positions des maxima et des minima d'amplitude relatifs sur l'échelle des longueurs d'ondes.

**17.** Dispositif selon l'une quelconque des revendications 11 à 16, caractérisé en ce qu'on calcule en ligne les rapports des amplitudes des maxima, des minima et entre les maxima et les minima.

**18.** Dispositif selon l'une quelconque des revendications 11 à 17, caractérisé en ce que, pour l'identification et la distinction, on part des largeurs de demi-amplitudes (22, 23), respectivement des largeurs de base (24) des spectres (1, 2, 5, 6).

**19.** Dispositif selon l'une quelconque des revendications 11 à 18, caractérisé en ce qu'on fait la somme des quotients (39, 40) entre les valeurs de coordonnées (37, 38, 41, 42) correspondantes des spectres mesurés en ligne in situ et des spectres (1, 2, 5, 6) se trouvant dans la mémoire, et la substance qui présente la plus grande valeur cumulée est considérée comme étant reconnue.

**20.** Dispositif selon l'une quelconque des revendications 11 à 19, caractérisé en ce qu'on sélectionne un premier seuil (3, 8) pour l'amplitude maximale en dessous duquel les constituants des récipients/bouteilles restent dans la ligne de transvasement, tandis que des substances identiques quant à leur contenu sont guidées vers un éclusage au-dehors dès qu'un second seuil (4, 7) qui se situe au-dessus du premier est dépassé.

_Fig. 1_

Extinktion E

Wellenlänge

Fig. 2

Extinktion E

Wellenlänge

S_4

S_3

EP 0 557 814 B1

*Fig. 3*

Extinktion E

Wellenlänge

EP 0 557 814 B1

12

## Fig. 4

Lemonenspektrum

## Fig.5a
## Quotientenspektum

39

— Quotient  ▨ Azeton 38  ☐ Benzol 37

unterschiedliche Spektren

## Fig.5b
## Quotientenspektrum

40

— Quotient  ▨ Spektrum A 41  ☐ Spektrum B 42

14

Fig 6